Europäisches Patentamt

European Patent Office

Office européen des brevets

(19)

(11) Veröffentlichungsnummer : **0 348 443 B1**

(12) # EUROPÄISCHE PATENTSCHRIFT

(45) Veröffentlichungstag der Patentschrift :
22.07.92 Patentblatt 92/30

(51) Int. Cl.$^5$ : **G01N 33/36**

(21) Anmeldenummer : **88908127.9**

(22) Anmeldetag : **28.09.88**

(86) Internationale Anmeldenummer :
**PCT/CH88/00175**

(87) Internationale Veröffentlichungsnummer :
**WO 89/03531 20.04.89 Gazette 89/09**

---

(54) VORRICHTUNG ZUR AUTOMATISCHEN FEINHEITSBESTIMMUNG VON TEXTILEM PRÜFGUT, UND VERWENDUNG DER VORRICHTUNG.

---

(30) Priorität : **06.10.87 CH 3907/87**

(43) Veröffentlichungstag der Anmeldung :
**03.01.90 Patentblatt 90/01**

(45) Bekanntmachung des Hinweises auf die Patenterteilung :
**22.07.92 Patentblatt 92/30**

(84) Benannte Vertragsstaaten :
**AT BE CH DE FR GB IT LI**

(56) Entgegenhaltungen :
**EP-A- 0 129 076**
**DE-A- 1 940 291**
**DE-A- 2 152 935**
**FR-A- 2 556 747**
**US-A- 4 084 434**

(73) Patentinhaber : **ZELLWEGER USTER AG**
**Wilstrasse 11**
**CH-8610 Uster (CH)**

(72) Erfinder : **HEUSSER, Eduard**
**Heinrichstrasse 17**
**CH-8610 Uster (CH)**

EP 0 348 443 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung betrifft eine Vorrichtung zur automatischen Feinheitsbestimmung von textilem Prüfgut in Form von Garnen, Vorgarnen oder Bändern, mit einer Einrichtung zum Abzug definierter Prüfgutlängen von einem Vorrat, mit einer Waage, mit einer Auswerteeinheit, mit im Prüfgutlauf vor der Waage angeordneten Mitteln zur Formung des jeweils abgezogenen Prüfguts zu einem Paket, und mit Mitteln zur Ablage des Pakets auf der Waage.

Derartige Feinheitsbestimmungen sind in der textilen Praxis sehr wichtig, weil Schwankungen der Feinheit (auch als Nummerschwankungen bezeichnet), das sind Schwankungen der längenbezogenen Masse beispielsweise eines Garns, zu Schussstreifen in Geweben und Ringeln in Maschenware führen. Um diese Qualitätseinbussen möglichst zu vermeiden, werden in den Textillabors von Spinnereien laufend Feinheitsbestimmungen oder Nummerkontrollen durchgeführt, damit Bedienungs- oder Einstellfehler möglichst rasch erkannt und behoben werden können.

Diese Kontrollen werden im Labor bisher halbautomatisch vorgenommen, indem die Prüfgutlängen mehr oder weniger manuell von einer Spule abgezogen und ebenfalls manuell auf die Wage gelegt werden; die Auswertung erfolgt dann automatisch. Wenn man bedenkt, dass andere in Textillabors verwendete Prüfanlagen, wie beispielsweise der USTER TESTER (USTER - eingetragenes Warenzeichen der Zellweger Uster AG) einige hundert Meter Garn pro Minute verarbeiten, dann wird klar, dass Feinheitsbestimmungen nur stichprobenartig gemacht und wegen ihrer vergleichsweise bescheidenen Garnverarbeitungskapazität nicht mit anderen Prüfanlagen, wie beispielsweise Gleichmässigkeitsprüfern, vernetzt werden können. Gerade diese Vernetzungsmöglichkeit wäre aber ein wesentlicher Schritt in Richtung auf ein automatisches Textillabor.

Aus der DE-A-1 940 291 ist eine Vorrichtung bekannt, bei der vom Vorrat abgezogene Faden entweder direkt auf die Waagschale geblasen und somit also nicht zu einem Paket geformt, oder aber in einen rohrförmigen Zwischenbehälter geblasen wird, wobei er sich zu einem Knäuel zusammenlegt. Dieser sogenannte Knäuel wird dann mittels Pressluft auf die Waagschale geblasen, welche die Form eines einseitig geschlossenen Rohres aufweist. Wie der Knäuel nach der Messung von der Waagschale, also aus dem Rohr heraus, entfernt wird, ist nicht ersichtlich.

Letzteres dürfte einer der Gründe sein, warum sich diese Vorrichtung in der Praxis nicht durchgesetzt hat. Ein anderer Grund liegt darin, dass auch trotz des "Knäuels" nicht ausgeschlossen werden kann, dass ein Teil des Fadens über den Rand der Waagschale hängt oder sich irgendwo verfängt, was das Messergebnis in nicht tolerierbarer Weise verfälschen würde.

Durch die Erfindung soll nun eine Vorrichtung zur Feinheitsbestimmung angegeben werden, deren Einsatz eine automatische Nummerkontrolle ermöglicht. Die Messergebnisse sollen exakt und zuverlässig sein, und die Leerung der Waagschale vom Prüfgut soll problemlos und störungsfrei funktionieren.

Diese Aufgabe wird erfindungsgemäss dadurch gelöst, dass die genannten Mittel zur Formung des Pakets durch ein Gehäuse mit einem glockenförmigen Oberteil und mit einem relativ zu diesem bewegbaren Bodenteil gebildet sind, wobei der glockenförmige Oberteil eine seitlich in diesen mündende, mit Druckluft beaufschlagte Transportleitung für das Prüfgut aufweist und an der Innenwand des Gehäuses die Formung des Prüfguts zu einem knäuelartigen Paket erfolgt, und dass Mittel zum Entfernen des knäuelartigen Pakets von der Waage nach der Messung vorgesehen sind.

Die Erfindung beruht auf der neuen Erkenntnis, dass die Ursache dafür, dass sich der bekannte Lösungsvorschlag bisher nicht durchsetzen konnte, darin besteht, dass bei diesem das Prüfgut entweder ohne irgendeine zusätzliche Manipulation oder aber über einen den Faden nur mehr oder weniger zusammenlegenden Zwischenbehälter in die Waagschale gefördert wird. Aufgrund dieser Erkenntnis wurde eine Lösung gefunden, die darin besteht, dass das Prüfgut vor der Ablage auf der Waagschale zu einem knäuelartigen Paket geformt wird. Dies garantiert, dass nicht Teile des Prüfguts über den Rand der Waagschale hängen oder sich irgendwa verfangen, und ausserdem kann der Knäuel nach der Messung einfach entfernt werden, sei dies durch Blasen oder durch ein mechanisches Organ.

Die Erfindung betrifft weiter eine Verwendung der genannten Vorrichtung in Kombination mit einem Gleichmässigkeitsprüfgerät. Diese Verwendung ist dadurch gekennzeichnet, dass die Vorrichtung über eine Transportleitung mit dem Gleichmässigkeitsprüfgerät verbunden und an dessen Auswerteeinheit angeschlossen ist.

Erfindungsgemäss sind also Gleichmässigkeitsprüfer und Vorrichtung zur Feinheitsbestimmung nicht mehr in einem gemeinsamen Gehäuse vereinigt. Gerade dadurch wird aber die weitere Automatisierung des Textillabors entscheidend verbessert.

Im folgenden wird die Erfindung anhand eines Ausführungsbeispiels und der Zeichungen näher erläutert; es zeigt:

Fig. 1 eine Perspektivdarstellung einer Garnprüfanlage mit einem Gleichmässigkeitsprüfer und einer Einrichtung zur Feinheitsbestimmung,

Fig. 2, 3 eine schematische Darstellung der Einrichtung zur Feinheitsbestimmung in zwei Ansichten,

Fig. 4, 5 ein Detail der Einrichtung zur Feinheitsbestimmung in zwei Ansichten,

Fig. 6 eine schematische Darstellung eines Details des Gleichmässigkeitsprüfers von Fig. 1; und

Fig. 7, 8 Diagramme zur Funktionserläuterung.

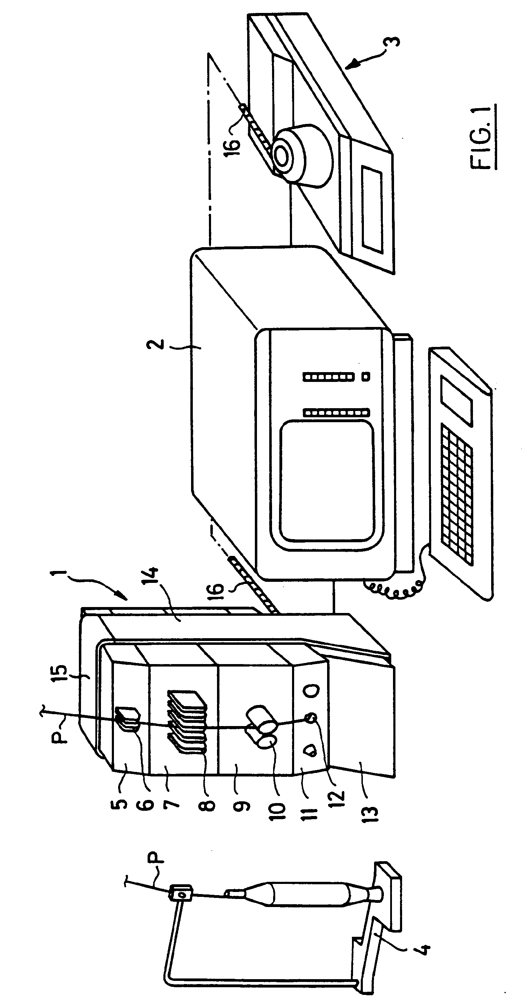

Die in Fig. 1 dargestellte Garnprüfanlage besteht aus einem Gleichmässigkeitsprüfer 1 zur Bestimmung der Masseschwankungen von textilem Prüfgut, wie Garnen, Vorgarnen oder Bändern aus Stapelfasern, aus einer Bedienungs/ Auswerteeinheit 2, aus einem Feinheitsprüfer 3 und aus einem Gestell 4 für die Aufmachungseinheiten mit dem Prüfgut P, das sind beispielsweise Garn- oder Vorgarnspulen. Garnprüfanlagen dieser Art sind mit Ausnahme des Fein heitsprüfers 3 bekannt und werden beispielsweise von der Anmelderin der vorliegenden Patentanmeldung unter der Bezeichnung USTER TESTER (USTER - eingetragenes Warenzeichen der Zellweger Uster AG) weltweit vertrieben.

Anstelle des dargestellten Gleichmässigkeitsprüfers 1 für Prüfgut aus Stapelfasern kann selbstverständlich auch ein solcher für Filamentgarne verwendet werden, der zwar im Aufbau etwas vom dargestellten Gleichmässigkeitsprüfer abweicht, prinzipiell aber mit diesem übereinstimmt. Es wird in diesem Zusammenhang auf die GB-A-2 192 722 verwiesen. In dieser Patentanmeldung ist auch gezeigt, dass die Garnprüfanlage üblicherweise noch eine Ausgabeeinheit in Form eines Druckers aufweist; dieser Drucker ist in Fig. 1 aus Platzgründen weggelassen, wird jedoch in den meisten Fällen vorhanden sein.

Der Gleichmässigkeitsprüfer 1 für das Prüfgut P besteht darstellungsgemäss aus mehreren Modulen, welche in Laufrichtung des Prüfguts P, das ist in der Figur von oben nach unten, wie folgt angeordnet sind: Zuerst ein Modul 5 mit einer Fadenführungseinrichtung 6, beispielsweise einer Fadenbremse, anschliessend ein Modul 7 mit einem Messorgan 8, anschliessend ein Modul 9 mit einer Vorschubeinrichtung 10 und anschliessend ein Modul 11 mit einer Absaugdüse 12. Das unterste Modul 11 ist auf einen Sockel 13 aufgesetzt, und alle genannten Module 5, 7, 9 und 11 sowie der Sockel 13 sind in einem Rahmen 14 mit einem bügelartigen Oberteil 15 angeordnet und von diesem Rahmen gehalten.

Das Messorgan 8, durch welches das Prüfgut P durch die durch ein Walzenpaar gebildete Vorschubeinrichtung 10 gezogen wird, ist ein sogenanntes kapazitives Messorgan. Dieses ist in den US Patenten 3 754 172 und 3 788 138 beschrieben, auf deren Offenbarung hiermit Bezug genommen wird. Die Vorschubeinrichtung 10, welche durch ein Paar von Transportwalzen gebildet ist, ist von dem bereits genannten USTER TESTER bekannt und wird hier nicht näher beschrieben. Detailliertere Beschreibungen von Teilen des Gleichmässigkeitsprüfers 1 sind in den Patentanmeldungen EP-A-266 611, EP-A-266 612, EP-A-266 614 und EP-A-266 615 zu finden.

Die Bedienungs-/Auswerteeinheit 2 enthält unter anderem einen Analog-/Digitalwandler und einen Rechner und ist darstellungsgemäss mit einem Bildschirm kombiniert. Die vom Messorgan 8 laufend erzeugten elektrischen Signale werden vom Rechner der Auswerteeinheit 2 verarbeitet und in geeigneter Form in einem in der Auswerteeinheit 2 integrierten Speicher gespeichert und können vor ihrem Ausdruck auf dem nicht dargestellten Drucker auf dem Bildschirm dargestellt werden. Dies hat den Vorteil, dass man alle anfallenden Daten vorerst auf dem Bildschirm zur Anzeige bringen und nur ausgewählte Daten für den Ausdruck auf dem Drucker bestimmen kann.

Die vom Messorgan 8 erzeugten Signale sind für den Prüfgutquerschnitt repräsentativ und werden in der Bedienungs-/Auswerteeinheit beispielsweise zu folgenden Kenngrössen verarbeitet: Spektrogramm (Wellenlängenspektrum der Masseschwankungen); Klassierung und Zählung von Extremstellen mit dem Imperfection Indicator; Variationskoeffizient und Längenvariationskurve. Alle diese Kenngrössen sind von dem schon genannten USTER TESTER her bekannt.

An die Absaugdüse 12 ist ein Schlauch oder eine Leitung 16 angeschlossen. Dieser führt zum Feinheitsprüfer 3 und transportiert somit unter der Wirkung von Druckluft das Prüfgut P nach erfolgter Gleichmässigkeitsprüfung zur Feinheitsprüfung. Da die Feinheit oder die Nummer eines Garns als Masse pro Längeneinheit (beispielsweise in Gramm pro Meter) angegeben wird, ist die Feinheitsbestimmung eine Bestimmung der Masse einer definierten Prüfgutlänge. Der Gleichmässigkeitsprüfer 1 verarbeitet und prüft bis einige hundert Meter Prüfgut pro Minute, wobei die pro Zeiteinheit durch die Transportwalzen 10 abgezogene und geförderte Länge an Prüfgut P durch die Drehzahl der Transportwalzen 10 gegeben ist. Ist anderseits diese Drehzahl bekannt, dann ist die geförderte Prüfgutlänge durch die Zeit bestimmt, während der Prüfgut gefördert wird.

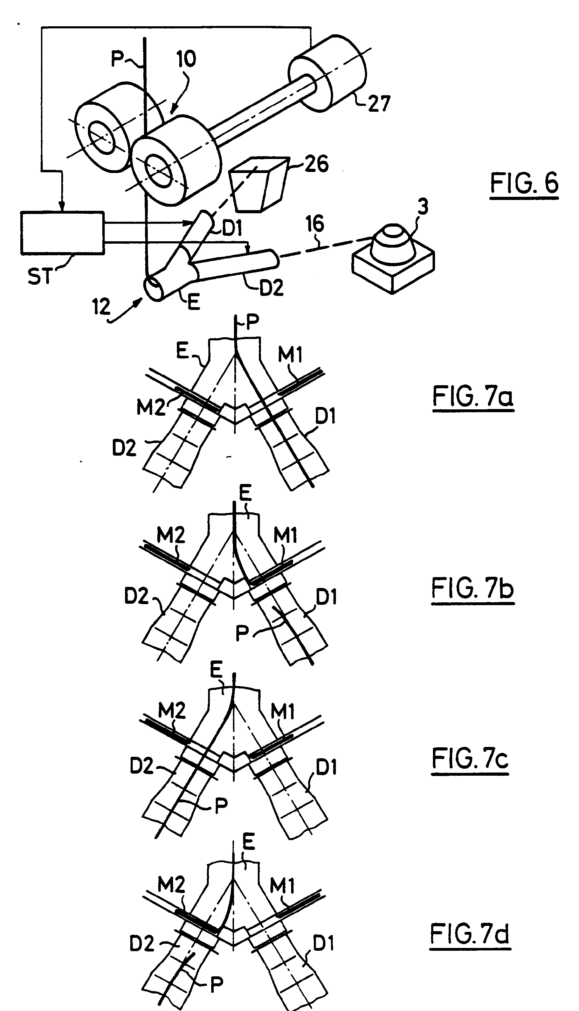

Somit braucht im Betrieb des Gleichmässigkeitsprüfers 1, wo die Fördergeschwindigkeit des Prüfguts P eingestellt wird und daher bekannt ist, lediglich über einen Zähler oder Zeitgeber ein Schneidorgan so angesteuert zu werden, dass das Prüfgut jeweils nach einer bestimmten Länge von beispielsweise 100 Metern abgeschnitten wird (siehe Fig. 7a-7d).

Die abgeschnittenen Prüfgutstücke werden durch den Schlauch 16 zum Feinheitsprüfer 3 geblasen und dort gewogen, und in der Bedienungs-/Auswerteeinheit wird die Feinheit errechnet, wobei verschiedenste Ergebnisse wie Einzelwerte, Mittelwerte, Gruppenwerte von Messreihen, Variationskoeffizienten und so weiter errechnet und angegeben werden können. Im Modul 11 ist eine Weiche mit Schneidorganen (Fig. 7a bis 7d) vorgesehen, durch welche die Prüfgutstücke wahlweise entweder zum Feinheitsprüfer 3 oder zu einem Abfallbehälter 26 (Fig. 6) geleitet werden.

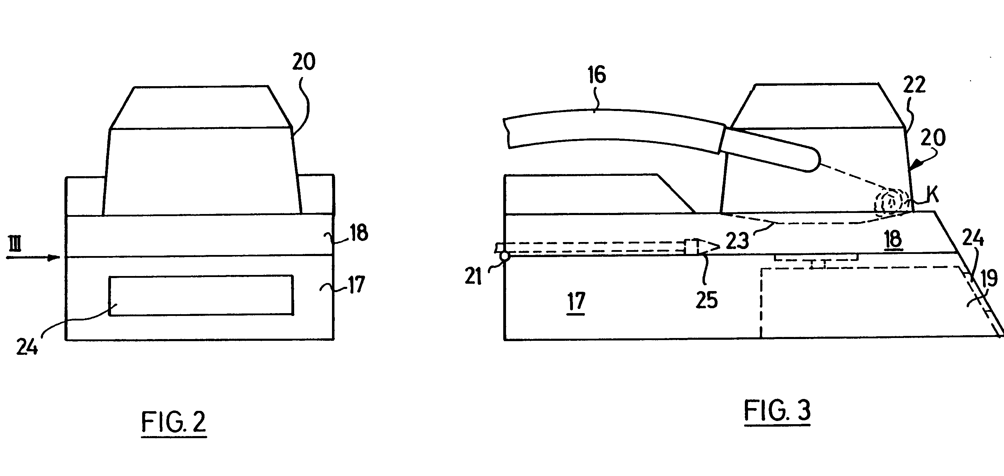

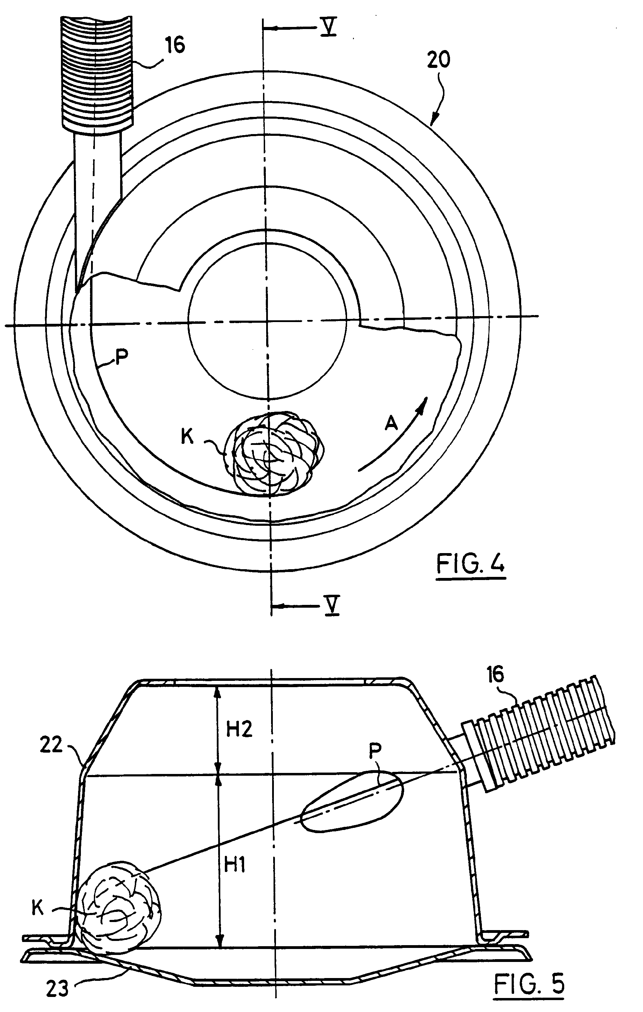

Der Feinheitsprüfer 3 ist in den Fig. 2 bis 5 ausführlich dargestellt. Dabei zeigt Fig. 2 eine Frontansicht und Fig. 3 eine Seitenansicht in Richtung des Pfeiles III von Fig. 2; Fig.4 zeigt eine teilweise aufgeschnittene Draufsicht auf ein Detail von Fig. 2, und Fig. 5 zeigt einen Schnitt nach der Linie V - V von Fig. 4.

Darstellungsgemäss besteht der Feinheitsprüfer 3 aus einem zweiteiligen Gehäuse mit einem Gehäuseunter- und einem Gehäuseoberteil 17 bzw. 18, mit einer im Gehäuseunterteil 17 eingebauten elektronischen Waage 19 und mit einem auf das Gehäuseoberteil 18 aufgesetzte glockenförmigen Behälter 20, in welchen die Leitung 16 (Fig. 1) mündet. Der Behälter 20 ist in den Figuren 4 und 5 im Detail dargestellt.

Die beiden Gehäuseteile 17 und 18 sind über eine Schwenkachse 21 miteinander verbunden, um welche der Oberteil 18 relativ zum Unterteil nach oben geschwenkt werden kann. Ein entsprechender Motor und Antrieb sind in der in Fig. 3 linken Hälfte (bezogen auf Fig.2 also in der hinteren Hälfte) des Gehäuses 17, 18 angeordnet.

Der Behälter 20 besteht aus einem oben offenen glockenförmigen Deckel 22 und einem tellerartigen, relativ zum Deckel 22 hubverstellbaren Boden 23. Für diese Hubverstellung, bei welcher der Boden 23 auf die Waage 19 abgesenkt wird, ist im Gehäuse 17, 18 ebenfalls ein spezieller Antriebsmechanismus (nicht dargestellt) vorgesehen.

Die Funktionsweise des Feinheitsprüfers 3 ist die folgende: Sobald die Feinheit eines Prüfguts bestimmt werden soll, wird dieses in der vorbestimmten Länge durch die Leitung 16 in den Behälter 20 geblasen und dabei aufgrund der Wirkung der Blasluft und der Form und Dimensionierung des Behälters 20 zu einem Knäuel K geformt. Nachdem die vorbestimmte Prüfgutlänge in den Behälter 20 gefördert wurde, liegt diese in Form des Knäuels K auf dem Boden 23. Nun wird der Boden 23 mit dem Knäuel K auf die austarierte Waage 19 abgesenkt. Die Waage 19 misst das Gewicht von Boden 23 und Knäuel, zieht das bekannte Gewicht vom Boden 23 ab und bringt das Nettogewicht des Knäuels in einem Anzeigefeld 24 zur Anzeige. Selbstverständlich wird das Gewicht auch an die Bedienungs-/Auswerteeinheit 2 zur Bestimmung der Feinheit und eventueller anderer davon abgeleiteter Parameter geliefert.

Sobald die Bestimmung des Gewichts des Knäuels beendet ist, das ist beispielsweise dann der Fall, wenn sich das von der Waage 19 gemessene Gewicht nicht mehr ändert, wird der Gehäuseoberteil 18 mit dem Deckel 22 um die Achse 21 nach oben geklappt, und zwar so weit, dass zwischen Deckel 22 und Boden 23 ein die Herausförderung des Knäuels K ermöglichender Spalt gebildet wird. Dann wird eine im Gehäuseunterteil 17 fest montierte Blasdüse 25 mit Druckluft beaufschlagt, wodurch der Knäuel K aus dem boden 23 heraus- und in einen Abfallbehälter (nicht dargestellt) hineingeblasen wird.

Wesentlich für die beschriebene Funktionsweise ist die Bildung des Knäuels K, die durch das Zusammenwirken der Blasluft in der Leitung 16 und des Behälters 20 erreicht wird. Selbstverständlich könnte der Transport des Prüfguts in der Leitung 16 anstatt mit Luft auch mit mechanischen Mitteln er folgen. Die anderen beschriebenen Funktionsschritte, wie das Absenken des Bodens 23 auf die Waage 19, das Aufklappen des Gehäuses 17, 18 und das Herausblasen des Knäuels K aus dem Boden 23 sind nur beispielhaft und nicht einschränkend zu verstehen.

Wie den Fig. 4 und 5 entnommen werden kann, hat der Deckel 22 des Gehäuses 20 eine glockenartige Form und besteht aus zwei kegelstumpfförmigen Teilen mit verschiedenen Neigungswinkeln. Die Wand des unteren Teils mit der Höhe H1 hat eine Neigung von 70° bis 90°, vorzugsweise etwa 85°, die Wand des oberen Teils mit der Höhe H2 hat eine Neigung von 45° bis 75°, vorzugsweise etwa 60°.

Die Höhe H1 ist etwa doppelt so gross wie H2. Am oberen Ende ist der Deckel 22 mit einer Oeffnung versehen, das untere, offene Ende ist bei der Bildung eines Knäuels K durch den Boden 23 geschlossen.

Die Leitung 16 mündet in den Deckel 22 in einer tangentialen Ebene unter einem spitzen Winkel von unter 45°, vorzugsweise von etwa 20° zur Horizontalen, wobei die Mündung etwas unterhalb des Uebergangsbereichs zwischen dem unteren und oberen Teil des Deckels 22 liegt. Das Prüfgut P wird von der Luft in der Leitung 16 gegen die Innenwand des unteren Deckelteils geblasen und gleitet dabei an dieser in Richtung des Pfeiles A (Fig. 4) entlang und spiralförmig nach unten. Sobald das Prüfgut P dabei auf dem Boden 23 auftritt, bildet sich ein Knäuel K.

Durch diesen Knäuel K wird nun die Bestimmung des Gewichts einer abgemessenen Länge und damit der Feinheit des Prüfguts im Unterschied zu bisher zuverlässig und reproduzierbar. Dieses Ergebnis ist für den Fachmann überraschend und nicht voraussehbar. Ausserdem kann der Knäuel nach der Gewichtsbestimmung einfach aus dem Gehäuse 20 entfernt werden (Fig. 3, Blasdüse 25). Damit ermöglicht der beschriebene Feinheitsprüfer 3 erstmals eine vollautomatische Feinheitsbestimmung auch bei hohen Prüfgeschwindigkeiten, was einen wesentlichen Schritt in Richtung auf das automatische Textillabor bedeutet.

Wenn auch im beschriebenen Ausführungsbeispiel der erfindungsgemässen Feinheitsprüfer in Verbindung mit einem Gleichmässigkeitsprüfer beschrieben ist, so ist seine Anwendung keineswegs auf diese Kombination beschränkt und ist in weiten Grenzen variabel. Wesentlich ist lediglich das Vorhandensein einer Abzugs- und Liefervorrichtung für definierte Prüflängen und einer an die Waage angeschlossenen Auswerteeinheit. Daraus wird ersichtlich, dass es sogar denkbar ist, den Feinheitsprüfer durch eine eigene Liefervorrichtung für das Prüfgut und ein eingebautes Auswertemodul zu einem autonomen Prüfgerät auszubauen.

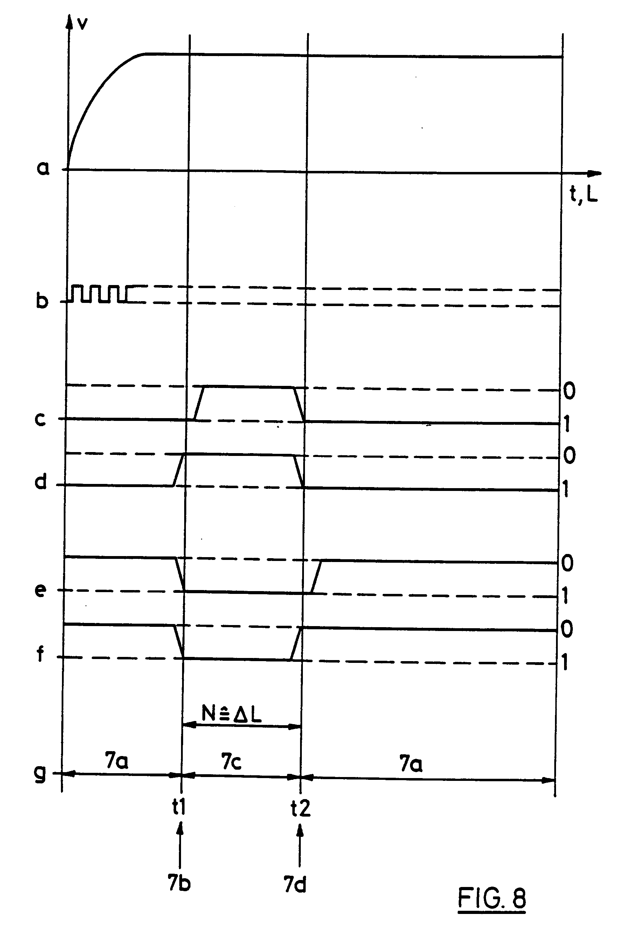

Fig. 6 zeigt schematisch das Walzenpaar der Vorschubeinrichtung 10 und die Absaugdüse 12 (Fig. 1), welche als Weiche für die wahlweise Weiterleitung des Prüfguts P zum Feinheitsprüfer 3 oder zu einem Abfallbehälter 26 ausgebildet ist. Die Fig. 7a bis 7d zeigen die Absaugdüse 12 in verschiedenen Betriebszuständen und Fig. 8 zeigt Diagramme zur Funktionserläuterung.

Darstellungsgemäss besteht die Absaugdüse 12 aus zwei an ein gemeinsames Einlaufstück E angeschlossenen Düsen D1 und D2, die vom Einlaufstück gabelförmig abzweigen und einzeln mit Druckluft beaufschlagbar sind. Diese Düsen sind vorzugsweise sogenannte Coanda-Düsen der in der FR-A-2 606 883 beschriebenen Art. Zwischen jeder Düse D1, D2 und dem Einlaufstück E ist ein steuerbares Messer M1 bzw. M2 angeordnet. An die Düse D1 ist eine zum Abfallbehälter 26 führende Leitung angeschlossen, an die Düse D2 die Leitung 16 zum Feinheitsprüfer 3. Die Steuerung der Düsen D1, D2 und der Messer M1, M2 erfolgt durch eine geeignete Steuerstufe ST anhand eines Tachometersignals, welches von einem mit einer der Walzen 10 formschlüssig verbundenen Geber 27 abgenommen wird. Das Tachometersignal ist in Fig. 8, Zeile b, dargestellt und besteht aus Rechteckimpulsen, deren Anzahl ein Mass der Länge des durch das Walzenpaar 10 abgezogenen Prüfguts P darstellt. Entsprechend ist in Fig. 8 auf der Abszisse sowohl die Zeit t als auch die abgezogene Prüfgutlänge L aufgetragen.

In Fig. 8 ist ausserdem in Zeile a die Geschwindigkeit v des Prüfguts eingezeichnet, in den Zeilen c und d die Ansteuerung von Düse D1 und Messer M1, in den Zeilen e und f die Ansteuerung von Düse D2 und Messer M2 und in Zeile g sind die den Diagrammen von Fig. 7 entsprechenden Betriebszustände markiert. In den Zeilen c und e bezeichnen die Symbole "0" und "1" die Zustände "Düse nicht in Betrieb" bzw. "Düse in Betrieb" und in den Zeilen d und f bezeichnen die Symbole "1" und "0" die Zustände "Messer offen" bzw. "Messer zu".

Da zu Beginn einer Messung eine bestimmte Zeit vergeht bis die Geschwindigkeit v des Prüfguts P den vorgegebenen Wert erreicht hat, wird bis zum Zeitpunkt t1, wo dies sicher der Fall ist, das Prüfgut in den Abfallbehälter 26 gefördert. Während dieser Zeitspanne (Zustand 7a in Zeile g von Fig. 8) ist gemäss den Fig. 7a und 8 die Düse D1 in Betrieb, die Düse 2 nicht in Betrieb, das Messer M1 ist offen und das Messer M2 ist zu. Zum Zeitpunkt t1 erfolgt durch das Messer M1 ein Schnitt (Fig. 7b), die Düse D2 wird aktiviert und das Messer M2 geöffnet. Die Düse D1 wird erst kurz nach t1 eingeschaltet, damit das abgeschnittene Prüfgut mit Sicherheit in den Abfallbehälter 26 gelangt.

Anschliessend bleibt das Messer M 1 zu und das Messer M2 offen, die Düse D1 ist nicht in Betrieb und die Düse D2 fördert das Prüfgut P zum Feinheitsprüfer 3. Dies entspricht Fig. 7c und dem Zustand 7c in Zeile g von Fig. 8.

Zum Zeitpunkt t2 (Zustand gemäss Fig. 7d) erfolgt nun ein Schnitt durch das Messer M2. Die Vorgänge laufen umgekehrt ab wie zum Zeitpunkt t1 und das abgeschnittene Prüfgut gelangt vollständig zum Feinheitsprüfer 3, wobei die Düse D2 erst kurz nach t2 abgeschaltet wird. Anschliessend ist wieder der Zustand gemäss Fig. 7a erreicht und das Prüfgut P wird bis zur nächsten Feinheitsbestimmung in den Abfallbehälter 26 gefördert. Die Dauer der Zeitspanne zwischen t1 und t2 ist durch eine einer vorgegebenen Prüfgutlänge Delta L von vorzugsweise 100 m entsprechende Anzahl N von Impulsen des Tachosignals (Fig. 8, Zeile b) definiert.

Da die Zeitspanne zwischen den Schnitten der Meser M1 und M2 bei konstanter Prüfgutgeschwindigkeit durch das Tachosignal genau bestimmbar ist, ergibt sich für die Feinheitsbestimmung eine exakte Prüfgutlänge und man kann eine Normlänge einfach auswählen.

## Patentansprüche

1. Vorrichtung zur automatischen Feinheitsbestimmung von textilem Prüfgut in Form von Garnen, Vorgarnen oder Bändern, mit einer Einrichtung (10) zum Abzug definierter Prüfgutlängen von einem Vorrat, mit einer Waage (19), mit einer Auswerteeinheit (2), mit im Prüfgutlauf vor der Waage (19) angeordneten Mitteln (20) zur Formung des jeweils abgezogenen Prüfguts (P) zu einem Paket (K), und mit Mitteln zur Ablage des Pakets auf der Waage (19), dadurch gekennzeichnet, dass die genannten Mittel zur Formung des Pakets (K) durch ein Gehäuse (20) mit einem glockenförmigen Oberteil (22) und mit einem relativ zu diesem bewegbaren Bodenteil (23) gebildet sind, wobei der glockenförmige Oberteil (22) eine seitlich in diesen mündende, mit Druckluft beaufschlagte Transportleitung (16) für das Prüfgut (P) aufweist und an der Innenwand des Gehäuses (20) die Formung des Prüfguts zu einem knäuelartigen Paket erfolgt, und dass Mittel (25) zum Entfernen des knäuelartigen Pakets von der Waage nach der Messung vorgesehen sind.

2. Vorrichtung nach Anspruch 1, dadurch gekennzeichnet, dass die Transportleitung (16) tangential in den glockenförmigen Oberteil (22) des Gehäuses (20) mündet.

3. Vorrichtung nach Anspruch 2, dadurch gekennzeichnet, dass der Bodenteil (23) oberhalb der Waage (19) angeordnet und tellerartig und auf die Waage absenkbar ausgebildet ist.

4. Vorrichtung nach Anspruch 3, dadurch gekennzeichnet, dass die Mittel zum Entfernen des knäuelartigen Pakets (K) von der Waage (19) durch eine im Niveau des Bodenteils (23) in dessen abgesenktem Zustand angeordnete Blasdüse (25) gebildet sind.

5. Vorrichtung nach Anspruch 2, dadurch gekennzeichnet, dass der glockenförmige Oberteil (22) aus zwei ineinandergehenden kegelstumpfförmigen Teilen gebildet ist, deren Höhen (H1, H2) sich etwa wie 2:1 und deren Neigungswinkel sich etwa wie 1:1,4 verhalten.

6. Vorrichtung nach Anspruch 5, dadurch gekennzeichnet, dass die Transportleitung 16 für das Prüfgut (P) im Übergangsbereich der beiden kegelstumpfförmigen Teile in den glockenförmigen Oberteil (22) mündet.

7. Vorrichtung nach Anspruch 6, dadurch gekennzeichnet, dass die Achse der Transportleitung (16) im Bereich der Einmündung in den Oberteil (22) schräg nach unten verläuft und gegen den Übergangsbereich zwischen Oberteil und Boden (23) des Gehäuses (20) gerichtet ist.

8. Verwendung der Vorrichtung nach Anspruch 1 in Kombination mit einem Gleichmässigkeitsprüfgerät, dadurch gekennzeichnet, dass die Vorrichtung (3) über eine Transportleitung (16) mit dem Gleichmässigkeitsprüfgerät (1) verbunden ist und mit diesem eine gemeinsame Auswerteeinheit (2) aufweist.

9. Verwendung nach Anspruch 8, dadurch gekennzeichnet, dass das Gleichmässigkeitsprüfgerät (1) eine Vorschubeinrichtung (10) und eine Absaugdüse (12) für das Prüfgut (P) aufweist, und dass die Transportleitung (16) an die letztere angeschlossen ist.

10. Verwendung nach Anspruch 9, gekennzeichnet durch eine Weiche zur wahlweisen Weiterleitung des Prüfguts (P) zur Transportleitung (16) oder zu einer anderen Leitung oder zu einem Behälter (26).

11. Verwendung nach Anspruch 10, dadurch gekennzeichnet, dass die Absaugdüse (12) als Weiche ausgebildet ist und aus zwei Düsen (D1, D2) besteht, denen ein gemeinsamer Einlaufteil (E) zugeordnet ist.

12. Verwendung nach Anspruch 11, dadurch gekennzeichnet, dass jeder Düse (D1, D2) ein Schneidorgan (M1 bzw. M2) zum Abtrennen des Prüfguts (P) zugeordnet ist, und dass die Schneidorgane (M1, M2) zwischen dem gemeinsamen Einlaufteil (E) und den Düsen (D1, D2) angeordnet sind.

13. Verwendung nach Anspruch 12, dadurch gekennzeichnet, dass zur Erzielung einer definierten Länge des Prüfguts (P) für die Feinheitsbestimmung die Düsen (D1, D2) und die Schneidorgane (M1, M2) gesteuert betätigbar sind, wobei die Steuerung anhand eines von der Vorschubeinrichtung (10) abgenommenen, für die Geschwindigkeit (v) des Prüfguts repräsentativen Tachosignals erfolgt.

## Claims

1. Apparatus for automatically determining the fineness of a textile sample in the form of yarns, rovings or slivers, having a device (10) for drawing off defined sample lengths from a supply, having a balance (19), having an evaluation unit (2), having means (20) disposed upstream of the balance (19) in the sample running direction for forming each drawn-off sample (P) into a package (K), and having means for depositing the package on the balance (19), characterized in that said means for forming the package (K) are formed by a housing (20) having a bell-shaped top part (22) and a bottom part (23) which is movable relative to said top part, with the bell-shaped top part (22) having, opening laterally therein, a compressed air operated transport line (16) for the sample (P) and with forming of the sample into a ball-like package being effected at the inner wall of the housing (20), and that means (25) are provided for removal after measurement of the ball-like package from the balance.

2. Apparatus according to claim 1, characterized in that the transport line (16) opens out tangentially into the bell-shaped top part (22) of the housing (20).

3. Apparatus according to claim 2, characterized in that the bottom part (23) is disposed above the balance (19) and is designed so as to be plate-like and capable of lowering onto the balance.

4. Apparatus according to claim 3, characterized in that the means for removing the ball-like package (K) from the balance (19) are formed by a blast nozzle (25) disposed at the level of the bottom part (23) in the latter's lowered state.

5. Apparatus according to claim 2, characterized in that the bell-shaped top part (22) is formed by two truncated cone-shaped parts verging one into the other, of which the ratio of the heights (H1, H2) is approximately 2:1 and the ratio of the angles of inclination is approximately 1:1.4.

6. Apparatus according to claim 5, characterized in that the transport line (16) for the sample (P) opens out into the bell-shaped top part (22) in the transition region of the two truncated cone-shaped parts.

7. Apparatus according to claim 6, characterized in that the axis of the transport line (16) extends obliquely downwards in the region of the point of entry into the top part (22) and is directed towards the transition region between top part and bottom (23) of the housing (20).

8. Use of the apparatus according to claim 1 in combination with a regularity test instrument, characterized in that the apparatus (3) is connected by a transport line (16) to the regularity test instrument (1) and has with said instrument a common evaluation unit (2).

9. Use according to claim 8, characterized in that the regularity test instrument (1) has a feed device (10) and an extraction nozzle (12) for the sample (P), and that the transport line (16) is connected to said extraction nozzle.

10. Use according to claim 9, characterized by a distributing guide for selectively passing the sample (P) on to the transport line (16) or to another line or to a receptacle (26).

11. Use according to claim 10, characterized in that the extraction nozzle (12) takes the form of a distributing guide and comprises two nozzles (D1, D2), with which a common run-in part (E) is associated.

12. Use according to claim 11, characterized in that a cutting element (M1 or M2) for severing the sample (P) is associated with each nozzle (D1, D2), and that the cutting elements (M1, M2) are disposed between the common run-in part (E) and the nozzles (D1, D2).

13. Use according to claim 12, characterized in that, to achieve a defined length of the sample (P) for determining fineness, the nozzles (D1, D2) and the cutting elements (M1, M2) are actuable in a controlled manner, with control being effected with the aid of a tacho signal which is removed from the feed device (10) and is representative of the velocity (v) of the sample.

## Revendications

1. Dispositif pour déterminer automatiquement la finesse d'un échantillon de textile sous la forme de filés, de mèches de préparation ou de rubans, comprenant une installation (10) pour dérouler des longueurs définies d'échantillon d'un organe d'alimentation, comportant une balance (19), une unité d'évaluation (2), des moyens (20) placés devant la balance (19) dans le sens d'avancement de l'échantillon pour conformer l'échantillon (P) déroulé respectivement en un paquet (K), et des moyens pour déposer le paquet sur la balance (19), caractérisé en ce que lesdits moyens pour former le paquet (K) sont constitués par un boîtier (20) possédant une partie supérieure (22) en forme de cloche et une partie de fond pouvant être déplacée par rapport à celle-ci, la partie supérieure (22) en forme de cloche présentant un conduit de transport(16) débouchant latéralement dans celle-ci, alimenté en air comprimé, pour l'échantillon (P) et que, sur la paroi interne du boîtier (20), est effectuée la conformation de l'échantillon en un paquet sous forme de pelote, et en ce que des moyens (25) sont prévus pour retirer le paquet sous forme de pelote de la balance après la mesure.

2. Dispositif selon la revendication 1, caractérisé en ce que le conduit de transport (16) débouche tangentiellement dans la partie supérieure en forme de cloche (22) du boîtier (20).

3. Dispositif selon la revendication 2, caractérisé en ce que la partie de fond (23) est disposée au-dessus de la balance (19) et est réalisée sous forme d'assiette et pouvant être baissée sur la balance.

4. Dispositif selon la revendication 3, caractérisé en ce que les moyens pour retirer le paquet sous forme de pelote (K) de la balance (19) sont constitués par une buse soufflante (25) disposée au niveau de la partie de fond (23) dans l'état abaissé de celle-ci.

5. Dispositif selon la revendication 2, caractérisé en ce que la partie supérieure (22) en forme de cloche est constituée de deux parties tronconiques qui rentrent l'une dans l'autre, dont les hauteurs (H1,H2) sont environ de 2:1 et leur angle d'inclinaison environ de 1:1,4.

6. Dispositif selon la revendication 5, caractérisé en ce que le conduit de transport (16) pour l'échantillon (P) débouche dans la partie supérieure (22) en forme de cloche dans la zone de transition des deux parties tronconiques.

7. Dispositif selon la revendication 6, caractérisé en ce que l'axe du conduit de transport (16), dans la zone de l'embouchure dans la partie supérieure (22), s'étend obliquement vers le bas et est dirigée contre la zone de transition entre la partie supérieure et le fond (23) du boîtier (20).

8. Utilisation du dispositif selon la revendication 1, en combinaison avec un appareil de vérification de régularité, caractérisée en ce que le dispositif (3) est relié par l'intermédiaire du conduit de transport (16) à l'appareil de vérification de régularité (1) et présente avec celui-ci une unité d'évaluation (2) commune.

9. Utilisation selon la revendication 8, caractérisé en ce que l'appareil de vérification de régularité (1) présente une installation d'avancement (10) et une buse d'aspiration (12) pour l'échantillon (P), et en ce que le conduit de transport (16) est raccordé à cette dernière.

10. Utilisation selon la revendication 9, caractérisée par un élément de séparation pour la transmission au choix de l'échantillon (P) au conduit de transport (16) ou à un autre conduit ou à un réservoir (26).

11. Utilisation selon la revendication 10, caractérisée en ce que la buse d'aspiration (12) est réalisée sous la forme d'élément de séparation et est constituée de deux buses (D1,D2), auxquelles,est associée une pièce d'introduction (E) commune.

12. Utilisation selon la revendication 11, caractérisée en ce qu'un organe de coupe (M1 ou M2) pour la séparation de l'échantillon (P) est associé à chaque buse (D1,D2), et en ce que les organes de coupe (M1,M2) sont disposés entre la pièce d'introduction commune (E) et les buses (D1,D2).

13. Utilisation selon la revendication 12, caractérisée en ce que pour obtenir une longueur définie de l'échantillon (P) pour la détermination de la finesse, les buses (D1,D2) et les organes de coupe (M1,M2) peuvent être actionnés de façon commandée, la commande étant effectuée à l'aide d'un signal tachymétrique reçu par l'installation d'avancement (10) et représentatif de la vitesse (v) de l'échantillon.

FIG.1

FIG. 3

FIG. 2

16

20

P

K

A

FIG. 4

V

V

22

H2

H1

P

16

K

23

FIG. 5

FIG. 6

FIG. 7a

FIG. 7b

FIG. 7c

FIG. 7d

FIG. 8